# EUROPEAN PATENT SPECIFICATION

(11) **EP 4 101 484 B1**
(45) Date of publication and mention of the grant of the patent: **26.11.2025**
(21) Application number: 21178697.5
(22) Date of filing: 10.06.2021
(51) Int. Cl.: A61M 5/20, A61M 5/315, A61M 5/31

(54) **DRUG DELIVERY DEVICE WITH DOSE COUNTER**
ARZNEIMITTELABGABEVORRICHTUNG MIT DOSISZÄHLER
DISPOSITIF D'ADMINISTRATION DE MÉDICAMENT AVEC COMPTEUR DE DOSE

(43) Date of publication of application: 14.12.2022
(73) Proprietor: Ypsomed AG, 3401 Burgdorf (CH)
(72) Inventor: SCHRUL, Christian, 3400 Burgdorf (CH); BOSSHARD, Simon Martin, 3006 Bern (CH); SCHEURER, Simon, 3006 Bern (CH); BRÜGGER, Martin, 3065 Bolligen (CH)
(74) Representative: Meier Obertüfer, Jürg

(56) References cited:
- WO-A1-2015/007816
- WO-A1-2016/055438

## Description

### Technical Field

The invention relates to a drug delivery device with a dose counter, the dose counter having a first unit wheel and a second unit wheel, the unit wheels being coupled by means of a gear which translates a continuous rotation movement of the first unit wheel into an intermittent rotation of the second unit wheel.

### Background Art

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens.

An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device.

There are several types of injection devices known in the art. Disposable injection devices are adapted to deliver medication from a container such as a pre-filled cartridge or syringe that is not intended to be replaced or refilled by the patient. Reusable, semi-disposable, or hybrid delivery devices have a components of the device that may be replaced by the patient, or a cartridge that may be refilled, while some components of the device may be reused with the replaced or refilled drug container.

Several doses may be administered with a single drug delivery device or with a single cartridge of a drug delivery device. In order to offer guidance to a patient whether the drug delivery device or cartridge may still be used or if a replacement is necessary, a drug delivery device may comprise a counter which shows the amount of medicament or of doses which have already been administered with or which remain in the drug delivery device or cartridge.

One convenient way of indication is to display a numerical value corresponding to the amount of medicament or of doses which remain or which have been administered. The use of a mechanical display is advantageous, as this allows the amount of medicament or doses remaining or already administered to be shown on manual drug delivery devices or on electronic drug delivery devices even when powered down.

Mechanical displays usually rely one or two wheels having numbers printed around their circumference, said wheel(s) being rotated to display an increasing or decreasing amount of medicament in the drug delivery device or cartridge. Such mechanical displays are also used in connection with dose setting mechanisms of drug delivery devices.

EP 2 983 771 B1 (Sanofi) discloses such a mechanical display in connection with a drug delivery device in the form of a pen-type injector. The mechanical display is used to display a dose set by a user of the injector. The mechanical display includes a first dose indicating member and a second dose indicating member, which are both rotatably supported on a longitudinal axis. Both dose indicating members have a sleeve-like shape with the same diameter and are both connected to a dose setting sleeve such as to visually indicate the size of the dose. Rotation of the dose setting sleeve is directly transferred to the first dose indicating member by means of a first display wheel. The dose setting sleeve comprises two oppositely located tapets which engage a second display wheel meshing with the second dose indicating member. Hence, the second dose indicating member is subject to two discrete, stepwise rotations during a complete revolution of the drive sleeve.

WO 2014/166916 A1 (Sanofi) describes a handheld injection device for selecting and dispensing a number of user variable doses of a medicament. The injection device comprises a number wheel which is suitable to display a dose set by a patient using a dose setting means. The dose setting means and the number wheel are coupled together by means of a gear such that the angle of rotation of the dose setting means differs from that of the number wheel. The gearing is provided by an epicyclic gearing. The injection device may comprise a second number wheel which is coupled to the first number wheel such that a continuous rotation of the first number wheel is translated into an intermittent motion of the second number wheel. The coupling may be realized by means of a Geneva drive.

EP 1 927 073 B1 (Aventis Pharma Ltd.) relates to a rotatably actuated counter mechanism usable with a dry powder inhaler. The counter displays to a user the number of doses remaining in the inhaler. The mechanism of the counter comprises a first and a second rotatable wheel each having a circumferential display surface thereon. The first and second wheels are connected with each other by means of a slave wheel. On a first face, the slave wheel comprises a gear which is engaged with gear teeth of the first rotatable wheel. On the other face, the slave wheel comprises a Geneva mechanism with a plurality of receptacles which may engage with a foot of the second rotatable wheel, such as to incrementally rotate the second rotatable wheel.

WO 2016/055438 A1 discloses a pen-injector including dial sleeve permanently rotationally and axially connected to a ring gear. The ring gear is coupled to a planet gear via a geared structure and the planet gear in turn meshes with a shaft of a first display member. The latter is coupled to a second display member by a gear which transmits a defined and limited rotation to the second display member. During dose dialing the first display member and depending on the size of dose and depending on the number of revolutions of the dose setting member, also the second display member starts to rotate to display consecutive rising dose numbers.

WO 2015/007816 A1 discloses an injection pen with an indication assembly for displaying a dose. The assembly includes a first display sleeve for a first digit and a second display sleeve for a second digit. The first and second display sleeve are decoupled from each other and each display sleeve is coupled to the drive mechanism of the injection pen by separate gear wheels.

### Summary of the invention

It is the object of the invention to create a drug delivery device with a counter mechanism which is small in dimension, reliable and which allows a cost-effective manufacture.

The solution of the invention is specified by the features of claim 1. According to the invention the drug delivery device includes a housing and a plunger rod with a threaded outer surface. The plunger rod is adapted to advance a piston inside a reservoir containing a medicament in order to dispense the medicament. The plunger rod is arranged parallel to and preferably congruent with a central axis of the housing. The drug delivery device further comprises a drive to move the plunger rod either in rotation or linearly about the central axis. Additionally, the drug delivery device comprises a dose counter to indicate the amount of medicament dispensed from or remaining in the reservoir, said dose counter comprising a first unit wheel and a second unit wheel both arranged next to each other along the central axis of the housing, the second unit wheel being coupled to the first unit wheel by means of a gear which translates a continuous rotation movement of the first unit wheel into an intermittent rotation of the second unit wheel. The first unit wheel comprises a spur wheel section on a first face, said spur wheel section having teeth which mesh with a spur wheel which is arranged concentric around the plunger rod, the spur wheel comprising at least one projection which meshes with the thread of the plunger rod.

By the provision of the spur wheel concentrically around the plunger rod, a simple and space saving transmission between the plunger rod and the dose counter is realized. As only a small number of parts is used for the dose counter, the reliability of the dose counter and of the drug delivery device is increased. Further, the assembly of the dose counter and of the drug delivery device is facilitated.

The drug delivery device preferably is an injection device. An injection device is removed from an injection site after each administration of a dose of a medicament. Alternatively, the drug delivery device may be an infusion device, i.e. a device with a cannula or needle which remains in the skin of a patient for a period of time, e.g. several hours or days in order to administer several doses of the medicament to the patient during the period of time. More preferably, however, the drug delivery device is a pen-shaped injection device.

The housing preferably is elongated and has a round, oval, rectangular or polygonal cross-section. The cross section may vary in size and form along the central axis of the housing. Preferably, the housing is made of a polymer material, more preferably of a medical grade polymer material, i.e. polymer material which fulfil the requirement of ISO 10993 and/or of the EU regulation 2017/745. The housing has a distal end and a proximal end. The distal end is the end of the housing which points towards a cannula or needle used to administer the medicament to a patient and which is connected to the drug delivery device. In the case that the drug delivery device is an injection device, the distal end of the housing will thus point towards the skin of a patient during an injection event. The proximal end of the housing therefore is the end of the housing which faces away of the needle or cannula. In the case that the drug delivery device is an injection device, the proximal end of the housing faces away of the skin of the patient during an injection event.

The housing is preferably open at its proximal and distal end such as to allow the assembly of the drug delivery device. The proximal and distal ends are closed with appropriate lids or the like in order to protect the elements of the drug delivery device located within the housing.

The drug delivery device preferably comprises a reservoir with a medicament. The reservoir may be located within the housing in certain embodiments. In this case, the drug delivery device is preferably discarded after all of the medicament in the reservoir has been administered to the patient. Preferably, however, the reservoir is included in a cartridge which is removably connectable with the housing, preferably at the distal end of the housing.

The reservoir comprises a piston which is movable in order to dispense the medicament through a cannula or needle. The cannula or needle is preferably connected or connectable to the distal end of the housing or of the cartridge.

The medicament preferably is a medicament used to treat diabetes. Preferably, the medicament is insulin or a derivative thereof.

The plunger rod is either moved in rotation or is moved linearly about the central axis by the drive. By means of movement of the plunger rod, the piston is moved within the reservoir in order to dispense the medicament. The drive preferably comprises an electric motor or a drive spring to impart a rotational or linear movement to the plunger rod. In this case, the drug delivery device is an automatic drug delivery device where the medicament is dispensed upon actuation of a button or the like by the patient. In the case that a drive spring is used, the drive spring may need to be tensioned or strained manually prior to dispensing the medicament. In an alternative embodiment, the drive may comprise an actuator which has to be manually moved by the patient in order to move the plunger rod. In this case, the drug delivery device is a manual drug delivery device. The plunger rod is preferably arranged such that its length axis is congruent with the central axis of the housing.

It is to be noted that the configuration of the dose counter may be also be used with another type of transmission between the plunger rod and the first unit wheel, i.e. without the spur wheel arranged concentrically around the plunger rod. However, the use of the spur wheel as described herein is preferred.

The spur wheel section of the first unit wheel is preferably unitary with the rest of the first unit wheel, i.e. the first unit wheel and the spur wheel section are configured as a mono-block. Alternatively, the spur wheel section may be a separate piece which is bonded to the first unit wheel, for example by means of an adhesive, by welding or by any other type of bonding which allows a permanent attachment.

Preferably, the first unit wheel and the second unit wheel have a peripheral surface onto which markings are arranged, preferably in the form of the numbers 0 to 9. Hence, preferably, the first unit wheel is indicative of the amount of medicament dispensed with or remaining in the drug delivery device or a cartridge connected therewith. Further preferably, the second unit wheel is indicative of every tenth unit or decade of units, such as like 10, 20, 30, 40 and so on, preferably up to 90, of the amount of medicament dispensed with or remaining in the drug delivery device or a cartridge connected therewith. Hence, the gear which translates the continuous rotation of the first unit wheel into an intermittent rotation of the second unit wheel is preferably configured such that the second unit wheel is rotated about one tenth of a full rotation for every full rotation of the first unit wheel. In a preferred embodiment, the gear may be in the form of a Geneva mechanism.

The peripheral surfaces of the first unit wheel and of the second unit wheel preferably have the same width. The housing preferably comprises an opening or window which allows to view at least a part of the peripheral surface of the first unit wheel and of the second unit wheel from outside of the housing. The first and second unit wheels are preferably made of a polymer material.

The units displayed on the first and second unit wheels may be volume units, such as ml or µl of the medicament dispensed from or remaining within the reservoir. Alternatively, the displayed units may be mass units such as mg or µg of the medicament contained in the volume dispensed from or remaining within the reservoir. Further alternatively, the units displayed may be percentage units relating to the total volume or amount of medicament dispensed from or remaining within the reservoir. Further alternatively, the displayed units may be units relating to an activity or biological equivalent, such as for example international units of insulin or any other arbitrary unit.

The spur wheel has the shape of a disc, i.e. the spur wheel has a central opening concentric with its axis of rotation. Further, the spur wheel comprises a first face and a second face as well as a multitude of teeth arranged around the circumference of the spur wheel. The spur wheel is dimensioned such that the plunger rod may be inserted through the central opening. Therefore, the spur wheel is concentrically arranged around the plunger rod. The spur wheel is arranged to be rotatable around the plunger rod but to be immobile in direction of the central axis. I.e. the spur wheel may not move along the plunger rod. The spur wheel preferably is made of a polymer material. Alternatively, the spur wheel may be made of a metal or metal alloy.

The spur wheel comprises at least one projection which meshes with the thread of the plunger rod. The thread may be an axially extending groove (deviation from rotational symmetry) in case of a rotating plunger rod configuration. The at least one projection preferably projects into the central opening of the spur wheel in order to mesh with the thread of the plunger rod. If the plunger rod is driven in rotation, the rotation is transmitted to the spur wheel by means of the interaction of an axial or longitudinal groove of the plunger rod with the at least one projection of the spur wheel. If the plunger rod is moved linearly (linear moving plunger rod configuration), the linear motion of the plunger rod is transformed into a rotational movement of the spur wheel by means of the interaction of the thread of the plunger rod with the at least one projection.

Preferably, the spur wheel comprises at least two projections which are arranged at an angular distance of 180° to each other.

The rotation of the spur wheel is transmitted to the first unit wheel by means of the meshing of the spur wheel with the spur wheel section of the first unit wheel. The spur wheel section is preferably arranged on a projection arranged on the first face of the first unit wheel.

In order to be accurate, the gear ratio between the plunger rod, the spur wheel and the spur wheel section of the first unit wheel is selected such that the rotation of the first unit wheel corresponds to the amount of the medicament dispensed from the reservoir by the movement of the plunger rod. This means, that the gear ratio depends on the size of the reservoir used in the drug delivery device or a cartridge connected thereto, the concentration of the medication (for example, Insulin U100 or U300) , as well as on the pitch of the thread of the plunger rod.

Preferably, the first gear is configured such that the direction of rotation of the second unit wheel is contrary to the rotation direction of the first unit wheel.

Preferably, the first unit wheel has a rotation axis which is parallel to the central axis and a diameter which is chosen such that the first unit wheel may be arranged besides the plunger rod within the housing.

Preferably, the second unit wheel is arranged concentric around the plunger rod. Hence, the second unit wheel has a disc shape with an opening which is concentric with the rotation axis of the plunger rod. The second unit wheel may be driven in rotation around the plunger rod. The rotation axis of the second unit wheel thereby is congruent with a length axis of the plunger rod. In the preferred case where the length axis of the plunger rod is congruent with the central axis of the housing, the rotation axis of the second unit wheel is therefore also congruent with the central axis of the housing.

Preferably, the diameter of the second unit wheel is larger than the diameter of the first unit wheel. Preferably, the second unit wheel has a diameter which is only slightly smaller than the diameter of an inner wall of the housing.

Preferably, the gear coupling the first unit wheel and the second unit wheel comprises one first drive cam arranged on a protrusion located on a second face of the first unit wheel, the first drive cam interacting with a ratchet wheel section of the second unit wheel, the ratchet wheel section including a multitude of teeth, preferably ten teeth.

The protrusion is located on the second face with is opposite of the first face which comprises the spur wheel section. Hence, the spur wheel is located on the other side of the first unit wheel as the protrusion. Preferably, the protrusion has the shape of a cylinder having a smaller diameter than the diameter of the first unit wheel. The protrusion is arranged on the rotation axis of the first unit wheel. The first drive cam preferably extends from the protrusion in an axial direction of the first unit wheel, i.e. in a direction which is perpendicular to the rotation axis of the first unit wheel.

The first drive cam and the ratchet wheel section are dimensioned and formed such that upon rotation of the first unit wheel the first drive cam may move into a gap between two adjacent teeth and subsequently interact with one of the both teeth which is in the direction of the rotation movement of the first unit wheel, such that said one tooth is being pushed by further rotation of the first drive cam, leading to a partial rotation of the ratchet wheel and hence of the second unit wheel. The teeth of the ratchet wheel section preferably have the form of a saw-tooth with one edge being at an acute angle in relation to the base surface of the ratchet wheel section and one edge being at approximately a right angle in relation to the base surface. The edge arranged at an acute angle is preferably on the side of the tooth which interacts with the first drive cam, i.e. the side of the tooth which points in a direction which is opposite to the direction of the rotation of the first unit wheel. The ratchet wheel section is preferably arranged on a first face of the second unit wheel, wherein the second face of the second unit wheel faces towards the second face of the first unit wheel.

As the first unit wheel comprises one first drive cam, the second unit wheel is rotated about a partial full revolution for every revolution of the first unit wheel. The partial revolution thereby is a fraction of the full revolution corresponding to the number of teeth of the ratchet wheel section. Hence, for example, in the preferred embodiment where the ratchet wheel section has ten teeth, the second unit wheel is turned about one tenth of a revolution for a full revolution of the first unit wheel.

Preferably, the second unit wheel and the ratchet wheel section are unitary, i.e. made of a single block of material. Alternatively, the ratchet wheel may however be a separate part which is permanently attached to the second unit wheel, e.g. by means of an adhesive, by welding or by any other type of permanent attachment.

With this configuration the movement of rotation of the first unit wheel into a first direction is translated into an intermittent rotation of the second unit wheel in the opposite, second direction. The second rotation direction of the second unit wheel is advantageous as the opposite rotation direction allows a reliable and robust translation of the rotation by the gear.

Preferably, the first unit wheel comprises a first blocking cam which is shaped such as to engage into one of multiple indentations of a first disc area of the second unit wheel when the first drive cam is not engaged with a tooth of the ratchet wheel section, and to not engage any indentation of the first disc area when the first drive cam engages a tooth of the ratchet wheel section.

This interaction between the first blocking cam of the first unit wheel with the indentations of the first disc area of the second unit wheel provides a means to secure the second unit wheel from rotating when there is no engagement between the first drive cam and any of the teeth of the ratchet wheel section.

Preferably, the protrusion of the first unit wheel comprises a first notch, wherein the section of the protrusion outside of the first notch forms the first blocking cam. The first notch is preferably located aside the first drive cam along the length of the protrusion and at an angular position which is chosen such that the first notch faces the first disc area when the first drive cam engages a tooth of the ratchet wheel section. The first notch preferably stretches over an area of less than 180° of the cross-section of the protrusion. The first notch preferably extends no further than the center of the protrusion in a radial direction, i.e. the first notch preferably is not deeper than the rotation axis of the first unit wheel.

The first notch is preferably arranged diametrically on the other side of the protrusion than the first drive cam. In this embodiment, the protrusion is hence arranged between the ratchet wheel section and the first disc area. The ratchet wheel section preferably is arranged in a direction towards the plunger rod and the first disc area towards an inner wall of the housing of the drug delivery device.

The second unit wheel preferably comprises a washer disc area next to the ratchet wheel section, the washer disc area being arranged opposite of the first disc area and of the first notch of the protrusion. The washer disc area and the first disc area are separated by a clearance. The clearance preferably has a width which corresponds to the diameter of the protrusion minus the extension or the depth of the first notch. With this arrangement the first notch is securely held, preferably free of clearance, between the washer disc area and the first disc area.

The indentations of the first disc area preferably have round edges or edges with a rounded section, the round edges or the rounded section having a radius which corresponds to the radius of the protrusion.

Preferably, the first unit wheel comprises a second blocking cam located at a distance from the first blocking cam on the protrusion, the second locking cam being shaped such as to engage into one of a multitude of indentations of a second disc area of the second unit wheel when the drive cam is not engaged with a tooth of the ratchet wheel section, and to not engage any indentation of the second disc area when the drive cam engages a tooth of the ratchet wheel section, wherein the first disc area and the second disc area are arranged such that the protrusion of the first unit wheel is located between the first disc area and the second disc area.

Hence, a second blocking action is provided in order to prevent the second unit wheel from rotating when the first drive cam is not engaged with a tooth of the ratchet wheel section. Preferably, the second locking cam is arranged adjacent to the first locking cam on the protrusion. This means that the second blocking cam is arranged at another point on the length axis of the protrusion. Provision of these two blocking actions has the advantage to ensure a blocking action of the second unit wheel in both rotation directions and at every angular position of the protrusion.

Preferably, the second blocking cam is arranged at a different angular position as the first blocking cam on the first unit wheel. Most preferably, the second blocking cam and the first blocking cam are spaced apart by 180°.

Preferably, the second blocking cam is configured as a second notch, wherein the section of the protrusion outside of the second notch forms the second blocking cam. The second notch is preferably located aside of the first notch along the length of the protrusion and at an angular position which is chosen such that the second notch faces the second disc area when the first drive cam engages a tooth of the ratchet wheel section. The second notch preferably stretches over an area of less than 180° of the cross-section of the protrusion. The second notch preferably extends no further than the center of the protrusion in a radial direction, i.e. the second notch preferably is not deeper than the rotation axis of the first unit wheel.

The dose counter preferably includes a third unit wheel which is arranged next to the second unit wheel and which is coupled to the second unit wheel by means of a second gear which allows a partial rotation of the third unit wheel for every full rotation of the second unit wheel, said partial rotation preferably being a tenth of a full rotation.

By means of the third unit wheel a three digit number may be used which is indicative of the amount of medicament dispensed with or remaining in the drug delivery device or a cartridge connected therewith.

Preferably, the third unit wheel comprises a peripheral surface onto which markings are arranged, preferably in the form of the numbers 0 to 9. Hence, the third unit wheel is indicative of every hundredth unit of the amount of medicament dispensed with or remaining in the drug delivery device or a cartridge connected therewith.

The second gear preferably is in the form of a Geneva mechanism.

The second gear preferably comprises a second drive cam arranged on the second unit wheel and a plurality of contact lugs, preferably ten contact lugs, arranged on a face of the third unit wheel in a paddle-wheel like arrangement, wherein the second drive cam is configured to engage between two neighbouring contact lugs once per revolution of the second unit wheel.

By the interaction of the single second drive cam with the plurality of contact lugs the third unit wheel is turned only a fraction of a full rotation for every rotation of the second unit wheel. The fraction corresponds to the number of contact lugs. I.e. with the preferred embodiment with three contact lugs, the third unit wheel is turned about a tenth of a full rotation for every rotation of the second unit wheel.

Each of the contact lugs protrudes from the face of the third unit wheel and has no contact with neighbouring contact lugs along their length. The contact lugs may be connected together by means of a ring-like structure at their free ends, i.e. their ends which are not connected with the face of the third unit wheel. With this configuration, a longitudinal element may be inserted through the paddle-wheel like arrangement.

With this configuration the intermittent rotation of the second unit wheel in the second direction is translated into an intermittent rotation of the third unit wheel in the opposite first direction. This means that the first and the third unit wheels both turn into the same first direction, while the second unit wheel, which is located between the first and the third unit wheel, turns in the opposite, second direction.

The second drive cam is preferably configured as a nose projecting in a radial direction of the second unit wheel. The second drive cam is preferably arrange on a second face of the second unit wheel. The second face of the second unit wheel preferably abuts on a first face of the third unit wheel, on which the paddle-wheel like arrangement of contact lugs is arranged.

Preferably, the second unit wheel includes a ring like protrusion with a gap, the gap being sized and positioned such that the ring like protrusion spans through the paddle-wheel like arrangement of contact lugs when the second drive cam is not engaged with any of the contact lugs and does not span through the paddle-wheel like arrangement when the second drive cam is engaged with a contact lug.

The ring like protrusion protrudes from the second face of the second unit wheel in an axial direction. The ring like protrusion extends through the paddle wheel like arrangement of contact lugs of the third unit wheel, wherein the ring like protrusion extends between two pairs of contact lugs arranged on opposite sides of the paddle wheel like structure. The ring like protrusion prevents the third unit wheel from rotating when it is engaged with the paddle wheel arrangement of contact lugs.

The third unit wheel may rotate when the gap reaches the paddle wheel like arrangement, i.e. when the ring like protrusion does not span through the paddle wheel like arrangement. The gap is arranged such that it reaches the paddle wheel like arrangement when the second drive cam engages with one of the contact lugs.

Preferably, a rotation axis of the third unit wheel is congruent with the rotation axis of the first unit wheel and the diameter of the third unit wheel corresponds to the diameter of the first unit wheel.

Hence, the third unit wheel is also arranged beside the plunger rod and between an inner wall of the housing and the plunger rod.

Preferably, the second unit wheel comprises an outer ring and a smaller concentric inner ring, the outer ring including a peripheral surface as well as the first disc area which is arranged on the backside of the peripheral surface, and wherein the inner ring comprises the ratchet wheel section, the second disc area, the second drive cam and the ring like protrusion arranged one to each other.

The inner ring further includes an opening which is sized to be arranged concentric about the plunger rod. Further, the second ring also comprises the washer disc area located between the ratchet wheel section and the second ring area.

The first and second ring are preferably connected with each other by means of at least one connection rib, preferably of at least two connection ribs. The first ring and the second ring preferably have the same width.

In a preferred embodiment the rotation axis of the first unit wheel is arranged in radial direction halfway (centrally) between an outer surface of the thread of the plunger rod and an inner surface of the housing. If the dose counter includes a third unit wheel it is preferably arranged radially halfway between the outer surface of the thread and the inner surface of the housing.

This arrangement allows an optimal translation of a movement of the first unit wheel to the second unit wheel by the gear as the rotation axis of the first unit wheel has a maximum possible distance to the rotation axis of the second unit wheel. The movement of the first unit wheel can thus translated in a reliable manner to the second unit wheel. Furthermore, the arrangement allows an optimal design of the size of the first and second unit wheel. Likewise, these adavantages apply for the third unit wheel.

Other advantageous embodiments and combinations of features come out from the detailed description below and the entirety of the claims.

### Brief description of the drawings

The drawings used to explain the embodiments show:
- Fig. 1: an exemplary embodiment of a drug delivery device according to the present invention;
- Fig. 2: a length section of the drug delivery device according to Fig. 1;
- Fig. 3: a perspective view of the dose counter of the drug delivery device according to Fig. 1 and 2;
- Fig. 4: a length section of the dose counter shown in Fig. 3;
- Fig. 5: a cross-section through the spur wheel of the dose counter according to Fig. 3;
- Fig. 6: a cross section through an embodiment of the gear between the first unit wheel and the second unit wheel;
- Fig. 7: a perspective view of the cross-section according to Fig. 6;
- Fig. 8: a second cross section of the gear as shown in Fig. 7 at a location which is located further distally of the first drive cam;
- Fig. 9: a third cross section of the gear according to Fig. 3 at a location which is located further distally of the first notch and of the first blocking cam;
- Fig. 10: the cross-section of Fig. 9 in a perspective view;
- Fig. 11: a cross-section through the second gear coupling the second unit wheel with the third unit wheel;
- Fig. 12: the cross-section of Fig. 11 in a perspective view;
- Fig. 13: a perspective view of the first unit wheel of the dose counter of Fig. 3;
- Fig. 14: a perspective view of the second unit wheel of the dose counter of Fig. 3 from a first face thereof;
- Fig. 15: a perspective view of the second unit wheel of the dose counter of Fig. 3 from a second face thereof;
- Fig. 16: shows a perspective view of the third unit wheel from a first face thereof.

In the figures, the same components are given the same reference symbols.

### Preferred embodiments

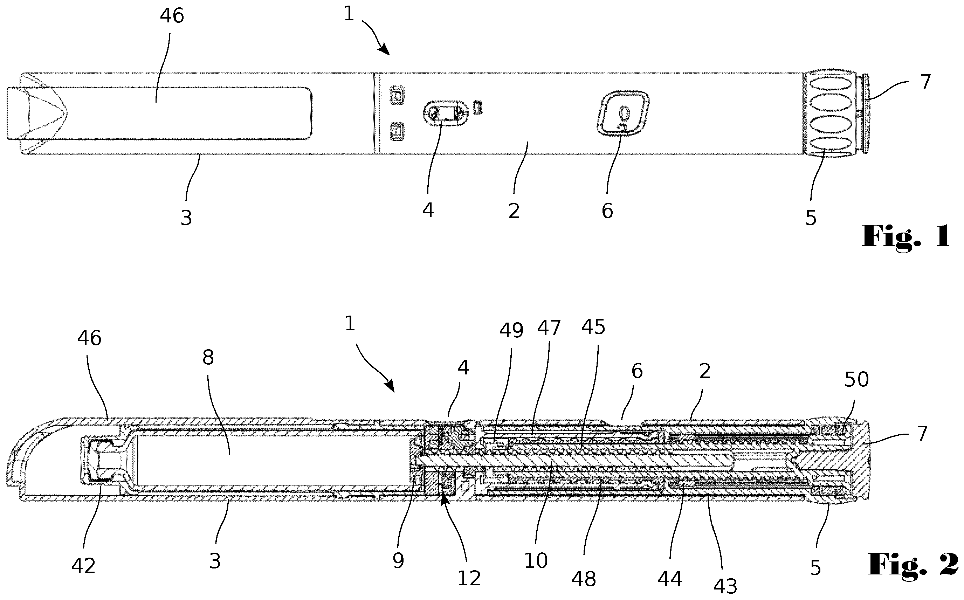

Fig. 1 shows an exemplary embodiment of a drug delivery device 1 according to the present invention. The drug delivery device 1 comprises a housing 2 with a generally round cross-section. At a distal end of the housing 2 a cartridge unit 3 containing a medicament is attached.

The device 1 depicted in the figures is a disposable injection pen. However, the dose counter and gear as descripted below may also implemented in a reusable device or a semi-disposable device including a disposable assembly with the cartridge and the medication and a reusable assembly with a drive mechanism.

The housing 2 of the device 1 includes a dose counter window 4. By means of the dose counter window 4 a patient using the drug delivery device 1 may read off the doses or amount of medicament dispensed with or remaining in the cartridge unit 3 which are indicated by means of a dose counter 12 integrated within the housing 2. Further, the housing 2 comprises a dispensing button 7 to dispense a defined dose of the medicament as well as a dose set knob 5 with which the dose volume may be selected. The selected dose volume is displayed in a dosage window 6 of the housing 2.

Fig. 2 is a length section of the drug delivery device shown in Fig. 1. The drug delivery device 1 is hereby turned by 90° upwards in the direction of view in comparison to the view shown in Fig. 1. Within the housing 2 a plunger rod 10 is arranged along a central axis of the housing 2. The plunger rod 10 is in engagement with the dose counter 12 and with a piston 9 slidably arranged within a reservoir filled with the medicament, when the cartridge unit 3 is attached to the housing 2. Sliding of the piston 9 in distal direction, i.e. to the left on Fig. 2 pushes the medicament out of the reservoir in order to dispense the medicament. The reservoir 8 comprises a thread 42 at its distal end in order to connect the reservoir 8 to a cannula or needle.

The dose set knob 5 is integral with dose sleeve 43. The dose sleeve 43 has the form of a hollow cylinder. On an outer surface of the dose sleeve 43 are helically arranged numbers which are visible through the dosage window 6 during dose setting and dispensing. In a distal portion the dose sleeve 43 comprises a dose insert 48 coaxially arranged within the dose sleeve 43. The dose insert 48 has a smaller diameter than the dose sleeve 43 and is fixedly connected or integral therewith. A cylindrical gap is present between an inner surface of the dose sleeve 43 and an outer surface of the dose insert 48. A holder 47 arranged within the housing 2 which is in the shape of a hollow cylinder at least partially protrudes into said gap. In the area of its distal end the dose insert 48 is in threading engagement with the holder 47.

In a proximal portion, the dose sleeve 43 has axially extending notches in which a stop nut 44 is in splined engagement. The stop nut 44 is guided in axial direction and is rotationally coupled to the dose sleeve 43 by this interaction.

The housing 2 further comprises a drive member 45 which is in the form of a hollow cylinder. In a distal portion the drive member 45 is concentrically arranged around the plunger rod 10, i.e. the plunger rod 10 is inserted within the drive member 45 from a distal end thereof. The plunger rod 10 is rotationally coupled to the drive member 45 but freely slidable therein in the axial direction. This may be achieved by the provision of at least one axial spline on the drive member 45 adapted to rotationally couple the drive member 45 with the plunger rod 10 but allowing a relative axial shift of the drive member 45 to the plunger rod 10. A proximal portion of the drive member 45 comprises an outer thread which is in threading engagement with the stop nut 44. At its proximal end the dispensing button 7 is inserted into the drive member 45. Further, the drive member 45 abuts on the dose sleeve 43 at its proximal end through coupling means 50.

To set a dose, a patient grabs the dose set knob 5 and rotates it. This also drives the dose sleeve 43 and the dose insert 48 into rotation. With this rotation, the dose sleeve 43 is screwed out of the housing 2, i.e. moved in the proximal (right direction in the figure) by means of the threading engagement of the dose insert 48 with the proximal end of the holder 47. This linear movement of the dose sleeve 48 entrains the linear movement of the drive member 45 in the proximal direction. However, the drive member 45 is not rotated as it is rotationally fixed by means of a retaining member 49 arranged on a distal end thereof. The retaining member 49 itself is slidable relative to the holder 47 but rotationally fixed thereto by means of splines. As the dose sleeve 43 is rotated but the drive member 45 is rotationally fix, the stop nut 44 moves up the outer thread of the proximal portion of the drive member 45.

Once the dose is set, the patient pushes the dispensing button 7 in the distal direction. As the dispensing button 7 is connected to the drive member 45 the distal movement of the dispensing button 7 is transferred to the drive member 45. This distal movement of the drive member 45 has two effects: Firstly, this movement creates a pressure on the coupling means which cause radial teeth arranged on the drive member 45 in the area of the coupling means with radial notches of the dose sleeve 43. Hence, the dose sleeve 43 and the drive member 45 are rotationally coupled. Secondly, said movement also leads to a disengagement of the rotational coupling between the drive member 45 and the retaining member 49 by a disengagement of protrusions located on the drive member 45 from notches located on the retaining member 49.

When the dispensing button 7 is further pushed distally, the dose sleeve 43 begins to rotate through the threading engagement of the dose insert 48 with the proximal end of the holder 47. As the dose sleeve 43 and the drive member 45 are now rotationally coupled by the coupling means 50, the drive member 45 is driven in rotation as well. By the rotational coupling of the plunger rod 10 with the drive member 45, the plunger rod 10 is also driven in rotation. As the plunger rod 10 is in threading engagement with the holder 27, the plunger rod 10 is screwed into the housing in the distal direction by this rotation. Hence, the plunger rod 10 may displace the piston 9 in the distal direction within the reservoir 8. Further, the retaining member 49 is also pushed in the distal direction.

Once the dose has been dispensed, i.e. the dose sleeve 43 has returned to its initial position as shown in Fig. 2, the patient stops pushing on the dispensing button 7. An elastic element located in the coupling means 50 then causes a slight movement of the drive member 45 relative to the dose sleeve 43 in the proximal direction, which leads coupling means 50 to release the rotational coupling of the drive member 45 with the dose sleeve 43. Further, this movement also leads to the re-engagement of the rotational coupling between the drive member 45 and the retaining member 49. The drug delivery device 1 is hence ready to dispense a further dose of the medicament.

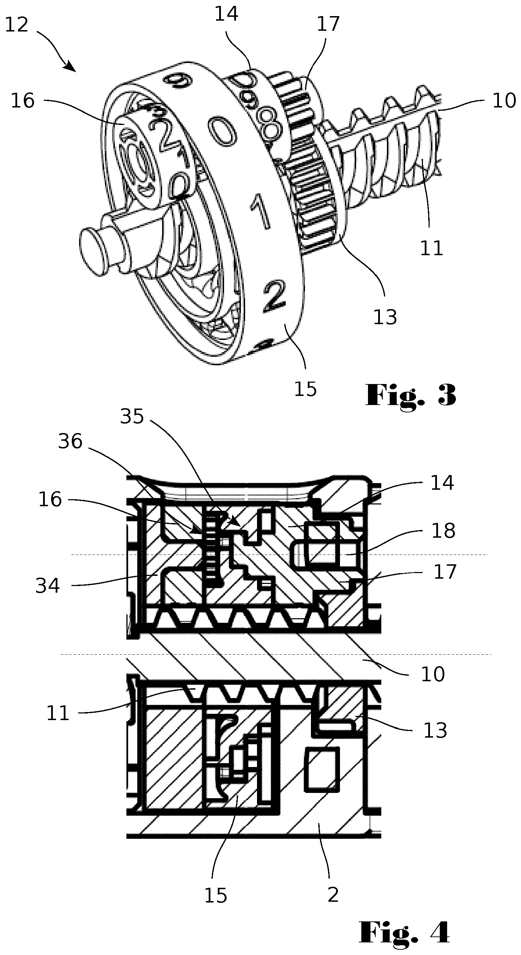

Fig. 3 is a perspective view of the dose counter 12 of the embodiment of the drug delivery device 1 shown in Figs. 1 and 2. Fig. 4 shows the dose counter 12 in a length section.

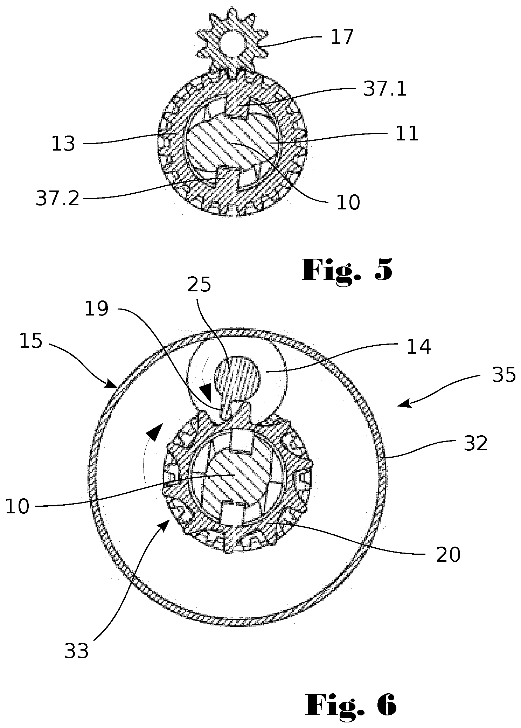

The dose counter 12 comprises a spur wheel 13 which is concentrically arranged around the plunger rod 10. The spur wheel 13 is splined to the plunger rod 10 by means of two projections 37.1, 37.2 (see Fig. 5). Hence, rotation and/or axial movement of the plunger rod 10 induces a rotation of the spur wheel 13. Further, the spur wheel 13 comprises teeth on an outer surface which mesh, i.e. are in meshing engagement with a spur gear section 17 located on a first face of a first dosage wheel 14. The first dosage wheel 14 has an outer circumference onto which numbers are printed or engraved. The first dosage wheel 14 has a diameter which is chosen such that the first dosage wheel 14 may be arranged between the plunger rod 10 and an inner wall of the housing 2. The rotation axis of the first dosage wheel 14 is parallel to the central axis of the housing 2 and hence also parallel to the extension of the plunger rod 10.

The dose counter 12 further comprises a second dosage wheel 15 which is concentrically arranged around the plunger rod 10 and besides the first unit wheel 14. The first dosage wheel 14 and the second dosage wheel 15 are coupled by a gear 35 (first geneva drive) which translates a continuous rotation movement of the first unit wheel 14 into an intermittent rotation of the second unit wheel 15. An embodiment of the gear will be explained further below. The second unit wheel 15 has an outer circumference with numbers printed or engraved thereon. The gear is configured such that the second unit wheel 15 rotates about one tenth of a revolution, i.e. 36° for each full rotation of the first gear wheel 14.

Additionally, the dose counter 12 includes a third dosage wheel 16. The third dosage wheel 16 has the same diameter as the first dosage wheel 14 and its rotation axis is congruent with the rotation axis of the first dosage wheel 14. The third dosage wheel 16 is arranged besides the second dosage wheel 15 and has an outer circumference with numbers printed or engraved thereon. The second dosage wheel 15 and the third dosage wheel 16 are coupled by means of a second gear 36 (second geneva drive). The second gear allows a partial rotation of the third unit wheel for every full rotation of the second unit wheel. The second gear is configured such that the third unit wheel 16 rotates about one tenth of a revolution, i.e. 36° for every full revolution of the second unit wheel 15. An embodiment of the gear will be explained further below.

The use of three dosage wheels 14, 15, 16 allows to display numbers from 000 to 999. However, it is also possible to use only the first unit wheel 14 for the dose counter 12. The second gear may alternatively be configured such that the second unit wheel is rotated about another fraction of a full revolution for every revolution of the first unit wheel 14, especially when the dose counter 12 only comprises the first unit wheel 14 and the second unit wheel 14. This allows to vary the number range which is displayed by the dose counter 12. The same applies to the second gear between the second unit wheel 15 and the third unit wheel 16.

As may be seen in Fig. 4, the first unit wheel 14 is rotationally supported by an axle bolt 18. The third unit wheel 16 is supported in rotation by an axle member 34.

Fig. 5 shows a cross section through the spur wheel 13. As may be seen, the spur wheel 13 is rotationally splined to the plunger rod 10 by means of a first projection 37.1 and a second projection 37.2. Rotation and/or axial movement of the plunger rod 10 hence leads to a rotation of the spur wheel 13. The spur wheel 13 comprises teeth on an outer surface which mesh with a spur wheel section 17 of the first unit wheel 14. The spur wheel 13 allows to implement a gear ratio between the plunger rod and the dose counter.

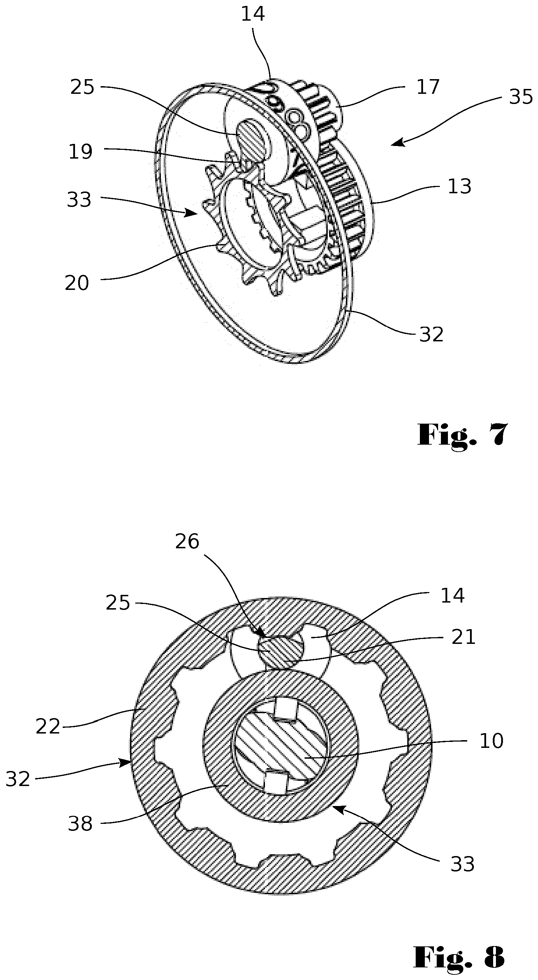

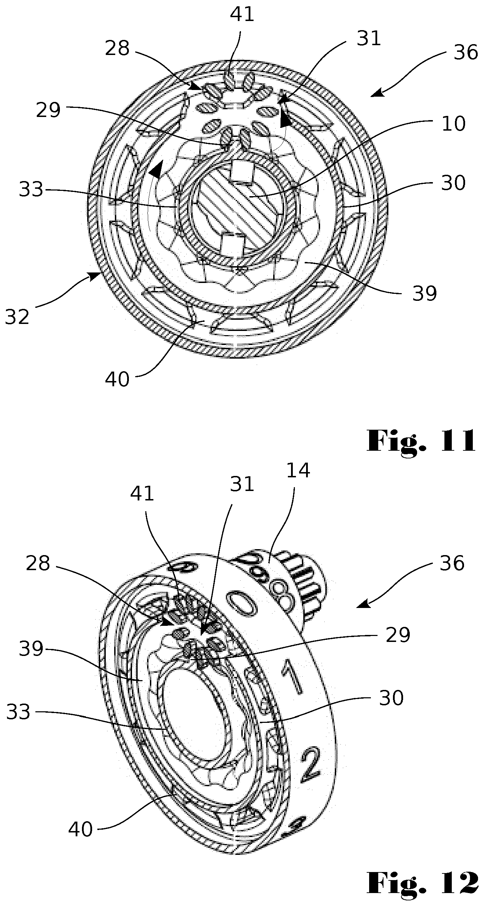

Fig. 6 is a representation of a cross section through an embodiment of the gear 35 between the first unit wheel 14 and the second unit wheel 15. Fig. 7 is a perspective view of the cross section according to Fig. 6. The first unit wheel 14 comprises a protrusion 25 in the form of a cylinder which extends from a second face of the first unit wheel 14. The protrusion 25 comprises a first drive cam 19 which radially extends from the protrusion 25. The first drive cam 19 interacts with a ratchet wheel section 20 of the second unit wheel 15. The ratchet wheel section 20 comprises ten teeth. The ratchet wheel section 20 is located on an inner ring 33 of the second unit wheel 15. The second unit wheel 15 further comprises an outer ring 32. Inner ring 33 and outer ring 32 are connected together by means of a multitude of connection ribs 40 (see Fig. 11, 12). As may be recognized in Fig. 6, the first drive cam 19 interacts with one of the teeth of the ratchet wheel section 20 for every revolution of the first unit wheel 13. The teeth of the ratchet wheel section 20 as well as the first drive cam 19 are formed such as to allow a smooth interaction between the first drive cam 19 and the teeth of the ratchet wheel section 20. With the shown embodiment, the second unit wheel 15 is rotated about a tenth of a full revolution for every full revolution of the first unit wheel 14 (direction of rotation indicated by arrows).

Fig. 8 shows a second cross section of the gear 35 at a location which is located further distally of the first drive cam 19. The protrusion 25 comprises a first notch 26. The area of the protrusion 25 outside of the first notch 26 constitutes a first blocking cam 21. The first blocking cam 21 may engage into recesses of a first disc area 22 of the second unit wheel 14. The first notch 26 and hence the first blocking cam 21 are arranged on the protrusion such that the first blocking cam 21 engages into a recess of the first disc area 22 when the first drive cam 19 is not engaged with any tooth of the ratchet wheel section 22. Hence, rotation of the second unit wheel 14 relative to the first unit wheel 13 is prevented. However, when the first drive cam 19 is engaged with a tooth of the ratchet wheel section 20, the notch 26 is oriented towards the first disc area 22. Therefore, the second unit wheel 14 may rotate relative to the first unit wheel 13. The first disc area 22 is arranged on the outer ring 32 of the second unit wheel 14 in the embodiment shown.

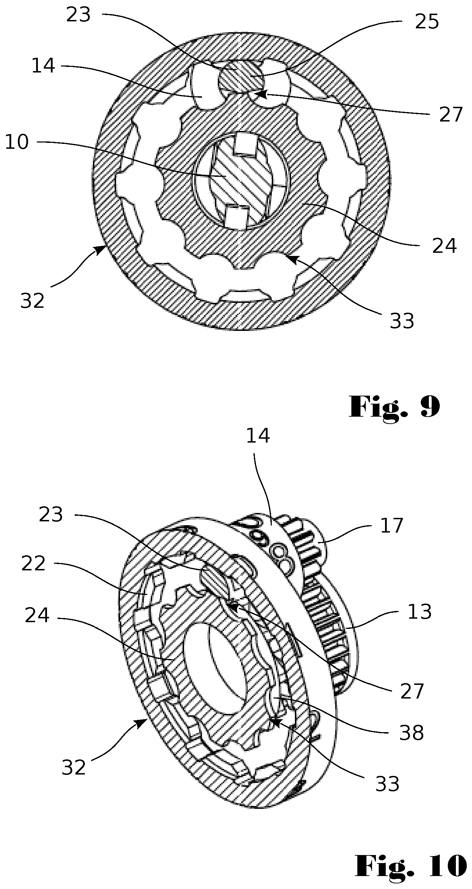

Fig. 9 shows a third cross section of the gear 35 at a location which is located further distally of the first notch 26 and of the first blocking cam 21. Fig. 10 shows the cross-section of Fig. 9 in a perspective view. The protrusion 25 comprises a second notch 27 which is located at another angular position than the first notch 26. In the embodiment shown, the first notch 26 and the second notch 27 are arranged 180° from each other. The area of the protrusion 25 outside of the second notch 27 constitutes a second blocking cam 23. The second blocking cam 23 may engage into recesses of a second disc area 24 of the second unit wheel 14. The second notch 27 and hence the second blocking cam 23 are arranged on the protrusion such that the second blocking cam 23 engages into a recess of the second disc area 24 when the first drive cam 19 is not engaged with any tooth of the ratchet wheel section 22. Hence, rotation of the second unit wheel 14 relative to the first unit wheel 13 is prevented. However, when the first drive cam 19 is engaged with a tooth of the ratchet wheel section 20, the second notch 27 is oriented towards the second disc area 24. Therefore, the second unit wheel 14 may rotate relative to the first unit wheel 13. The second disc area 24 is arranged on the inner ring 33 of the second unit wheel 14 in the embodiment shown. As may be seen on Fig. 10, the second disc area 24 is axially spaced apart from the ratchet wheel section 20 by a washer disc area 38.

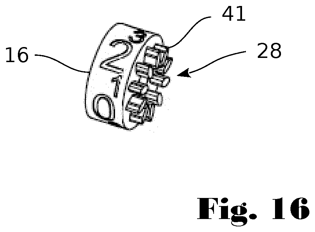

Fig. 11 shows a cross-section through the second gear 36 coupling and the second unit wheel 14 with the third unit wheel 16. Fig. 12 shows the cross-section of Fig. 11 in a perspective view. The third unit wheel 16 comprises a paddle-wheel like arrangement 28 of contact lugs 41 (see Fig. 16). The contact lugs 41 extend from a first face of the third unit wheel 16 in an axial direction. The paddle-wheel like arrangement 28 comprises ten contact lugs 41 in the embodiment shown. The second unit wheel 15 comprises a second drive cam 29 arranged on the inner ring 33. The second drive cam 29 protrudes from the inner ring 33 in an axial direction. The second drive cam 29 is configured to engage between two neighboring contact lugs 41 once per full revolution of the second unit wheel 15. Hence, the third unit wheel 16 is turned by one tenth of a full revolution for every full revolution of the second unit wheel 15.

The second unit wheel 15 comprises a ring like protrusion 30 with a gap 31. The ring like protrusion 30 protrudes in the axial direction from a support ring 39 which extends in a radial direction from the inner ring 33 of the second unit wheel 15. The ring like protrusion 30 is configured to span through the paddle-wheel like arrangement 28 of the third unit wheel 16 when the second drive cam 29 is not engaged between contact lugs 41 of the paddle-wheel like arrangement 28. This means that the ring like protrusion 30 extends through two pairs of neighboring contact lugs 41. Hence, the ring like protrusion 30 prevents a rotation of the third unit wheel 16 when the second drive cam 29 is not engaged between two neighboring contact lugs 41.

The gap 31 is located and dimensioned such as to be located at the paddle-wheel like arrangement 28 when the second drive cam 29 is engaged between contact lugs 41. Hence, the ring like protrusion does not prevent a rotation of the third unit wheel 16 in this case.

The inner ring 33 is connected to the outer ring 31 by a plurality of connection ribs 40 which are arranged between the outer ring 31 and the support ring 39.

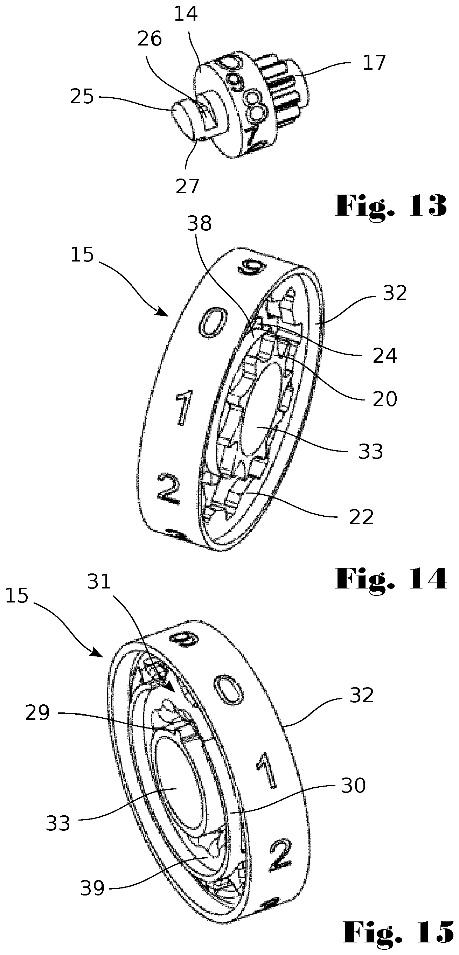

Fig. 13 is a perspective view of the first unit wheel 14 of the embodiment of the dose counter as described above. The spur gear section 17 is located on the first face of the first unit wheel 14. The protrusion 25 with the first notch 26 and the second notch 27 as well as the first drive cam 19 (not visible on Fig. 13) protrudes from the second face of the first unit wheel 14, which is on the opposite of the first face.

Fig. 14 shows a perspective view of the second unit wheel 15 from a first face thereof. As may be recognized in this figure, the second unit wheel 15 comprises an outer ring 32 as well as an inner ring 33. The ratchet wheel section 20 as well as the second disc area 24 are located on the inner ring 33, the second disc area 24 being spaced from the ratchet wheel section 20 in the axial direction by the washer disc area 38. The first disc area 22 is located on an inner wall of the outer ring 32. The first disc area 22 is located between the ratchet wheel section 20 and the second disc area 24 in the axial direction facing the washer disc area 38 of the inner ring 33.

Fig. 15 is a perspective view of the second unit wheel 15 from a second face thereof, the second face being opposite to the first face as shown in Fig. 14. As may be seen, the second drive cam 29 extends from the inner ring 33 in the radial direction. Further, the ring like protrusion 30, which extends in the axial direction from the support ring 39 is recognizable.

Fig. 16 shows a perspective view of the third unit wheel 16 from a first face thereof. The paddle-wheel like arrangement 28 with ten contact lugs 41 is well recognizable in Fig. 16.

### List of reference symbols

| | |
|---|---|
| 1 | Drug delivery device |
| 2 | Housing |
| 3 | Cartridge unit |
| 4 | Dose counter window |
| 5 | Dose set knob |
| 6 | Dosage window |
| 7 | Dispensing button |
| 8 | Reservoir |
| 9 | Piston |
| 10 | Plunger rod |
| 11 | Thread |
| 12 | Dose counter |
| 13 | Spur wheel |
| 14 | First unit wheel |
| 15 | Second unit wheel |
| 16 | Third unit wheel |
| 17 | Spur gear section |
| 18 | Axle bolt |
| 19 | First drive cam |
| 20 | Ratchet wheel section |
| 21 | First blocking cam |
| 22 | First disc area |
| 23 | Second blocking cam |
| 24 | Second disc area |
| 25 | Protrusion |
| 26 | First notch |
| 27 | Second notch |
| 28 | Paddle-wheel like arrangement |
| 29 | Second drive cam |
| 30 | Ring like protrusion |
| 31 | Gap |
| 32 | Outer ring |
| 33 | Inner ring |
| 34 | Axle member |
| 35 | Gear |
| 36 | Second gear |
| 37.1, 37.2 | Projection |
| 38 | Washer disc area |
| 39 | Support ring |
| 40 | Connection rib |
| 41 | Contact lug |
| 42 | Luer lock fitting |
| 43 | Dose sleeve |
| 44 | Stop nut |
| 45 | Drive member |
| 46 | Cap |
| 47 | Holder |
| 48 | Dose insert |
| 49 | Retaining member |
| 50 | Coupling means |

## Claims

1. A drug delivery device (1) including a housing (2), a plunger rod (10) with a threaded outer surface, the plunger rod (10) being adapted to advance a piston (9) inside a reservoir (8) containing a medicament in order to dispense the medicament, the plunger rod (10) being arranged parallel to and preferably congruent with a central axis of the housing (2), the drug delivery device (1) further comprising a drive (45) to move the plunger rod (10) either in rotation or linearly about the central axis,
wherein the drug delivery device (1) further comprises a dose counter to indicate the amount of medicament dispensed from or remaining in the reservoir (8), said dose counter comprising a first unit wheel (14) and a second unit wheel (15) both arranged next to each other along the central axis of the housing (2), the second unit wheel (15) being coupled to the first unit wheel (14) by means of a gear which translates a continuous rotation movement of the first unit wheel (14) into an intermittent rotation of the second unit wheel (15),
wherein the first unit wheel (14) comprises a spur wheel section on a first face, said spur wheel section (17) having teeth which mesh with a spur wheel (13) which is arranged concentric around the plunger rod (10),
**characterized in that** the spur wheel (13) comprises at least one projection (37.1) which meshes with a thread (11) of the plunger rod (10) to transform a rotation of the plunger rod (10) or a linear movement of the plunger rod (10) into a rotational movement of the spur wheel (13).

2. The drug delivery device (1) according to claim 1, **characterized in that** the first unit wheel (14) has a rotation axis which is parallel to the central axis and a diameter which is chosen such that the first unit wheel (14) may be arranged beside the plunger rod (10) within the housing (2).

3. The drug delivery device (1) according to any of claims 1 or 2, **characterized in that** the second unit wheel (15) is arranged concentric around the plunger rod (10).

4. The drug delivery device (1) according to any of claims 1 to 3, **characterized in that** the gear coupling the first unit wheel (14) and the second unit wheel (15) comprises one first drive cam (19) arranged on a protrusion (25) located on a second face of the first unit wheel (14), the first drive cam (14) interacting with a ratchet wheel section (20) of the second unit wheel (15), the ratchet wheel section (20) including a multitude of teeth, preferably ten teeth.

5. The drug delivery device according to claim 4, **characterized in that** the first unit wheel (14) comprises a first blocking cam (21) which is shaped such as to engage into one of multiple indentations of a first disc area (22) of the second unit wheel (15) when the first drive cam (19) is not engaged with a tooth of the ratchet wheel section (20), and to not engage any indentation of the first disc area (22) when the first drive cam (19) engages a tooth of the ratchet wheel section (20).

6. The drug delivery device (1) according to claim 5, **characterized in that** the first unit wheel (14) comprises a second blocking cam (23) located at a distance from the first blocking cam (21) on the protrusion (25), the second blocking cam (23) being shaped such as to engage into one of a multitude of indentations of a second disc area (24) of the second unit wheel (15) when the drive cam (19) is not engaged with a tooth of the ratchet wheel section (20), and to not engage any indentation of the second disc area (24) when the drive cam engages a tooth of the ratchet wheel section (20), wherein the first disc area (22) and the second disc area (24) are arranged such that the protrusion (25) of the first unit wheel (14) is located between the first disc area (22) and the second disc area (24).

7. The drug delivery device (1) according to any of claims 1 to 6, **characterized in that** the dose counter includes a third unit wheel (16) which is arranged next to the second unit wheel (15) and which is coupled to the second unit wheel (15) by means of a second gear (36) which allows a partial rotation of the third unit (16) wheel for every full rotation of the second unit wheel (15), said partial rotation preferably being a tenth of a full rotation.

8. The drug delivery device (1) according to claim 7, **characterized in that** the second gear (36) comprises a second drive cam (29) arranged on the second unit wheel (15) and a plurality of contact lugs (41), preferably ten contact lugs (41), arranged on a face of the third unit wheel (16) in a paddle-wheel like arrangement (28), wherein the second drive cam (29) is configured to engage between two neighbouring contact lugs (41) once per revolution of the second unit wheel (15).

9. The drug delivery device (1) according to claim 8, **characterized in that** the second unit wheel includes a ring like protrusion (30) with a gap (31), the gap being sized and positioned such that the ring like protrusion (30) spans through the paddle-wheel like arrangement (28) of contact lugs (41) when the second drive cam (29) is not engaged with any of the contact lugs (41) and does not span through the paddle-wheel like arrangement (28) when the second drive cam (29) is engaged with a neighbouring contact lug (41).

10. The drug delivery device (1) according to any of claims 7 to 9, **characterized in that** a rotation axis of the third unit wheel (16) is congruent with the rotation axis of the first unit wheel (14) and the diameter of the third unit wheel (16) corresponds to the diameter of the first unit wheel (14).

11. The drug delivery device (1) according to claim 10, **characterized in that** the second unit wheel (15) comprises an outer ring (32) and a smaller concentric inner ring (33), the outer ring (32) including a peripheral surface as well as the first disc area (22) which is arranged on the backside of the peripheral surface, and wherein the inner ring (33) comprises the ratchet wheel section (20), the second disc area, the second drive cam and the ring like protrusion (30) arranged one next to each other.

12. The drug delivery device (1) according to any of claims 1 to 11, **characterized in that** the rotation axis of the first unit wheel (14) is arranged in radial direction halfway between an outer surface of the thread (11) of the plunger rod (10) and an inner surface of the housing (2).

13. The drug delivery device (1) according to any of claims 1 to 12, **characterized in that** the device (1) is pen-shaped injection device.

## Patentansprüche

1. Arzneimittelabgabevorrichtung (1), die ein Gehäuse (2), eine Kolbenstange (10) mit einer mit einem Gewinde versehenen Außenoberfläche einschließt, wobei die Kolbenstange (10) geeignet ist, um einen Kolben (9) innerhalb eines Behälters (8), der ein Medikament enthält, vorzuschieben, um das Medikament auszugeben, wobei die Kolbenstange (10) parallel und vorzugsweise deckungsgleich zu einer Mittelachse des Gehäuses (2) angeordnet ist, die Arzneimittelabgabevorrichtung (1) ferner umfassend einen Antrieb (45), um die Kolbenstange (10) entweder drehend oder linear um die Mittelachse zu bewegen,
wobei die Arzneimittelabgabevorrichtung (1) ferner einen Dosiszähler umfasst, um die aus dem Behälter (8) ausgegebene oder darin verbleibende Medikamentenmenge anzuzeigen, der Dosiszähler umfassend ein erstes Einheitsrad (14) und ein zweites Einheitsrad (15), die beide nebeneinander entlang der Mittelachse des Gehäuses (2) angeordnet sind, wobei das zweite Einheitsrad (15) über ein Zahnrad mit dem ersten Einheitsrad (14) gekoppelt ist, das eine kontinuierliche Drehbewegung des ersten Einheitsrads (14) in eine intermittierende Drehung des zweiten Einheitsrads (15) umsetzt,
wobei das erste Einheitsrad (14) auf einer ersten Fläche einen Stirnradabschnitt umfasst,
wobei der Stirnradabschnitt (17) Zähne aufweist, die in ein Stirnrad (13) eingreifen, das um die Kolbenstange (10) konzentrisch angeordnet ist,
**dadurch gekennzeichnet, dass** das Stirnrad (13) mindestens einen Überstand (37.1) umfasst, der in ein Gewinde (11) der Kolbenstange (10) eingreift, um eine Drehung der Kolbenstange (10) oder eine lineare Bewegung der Kolbenstange (10) in eine Drehbewegung des Stirnrades (13) umzuwandeln.

2. Arzneimittelabgabevorrichtung (1) nach Anspruch 1,
**dadurch gekennzeichnet, dass** das erste Einheitsrad (14) eine Drehachse, die zu der Mittelachse parallel ist, und einen Durchmesser aufweist, der derart gewählt ist, dass das erste Einheitsrad (14) neben der Kolbenstange (10) innerhalb des Gehäuses (2) angeordnet werden kann.

3. Arzneimittelabgabevorrichtung (1) nach einem der Ansprüche 1 oder 2, **dadurch gekennzeichnet, dass** das zweite Einheitsrad (15) um die Kolbenstange (10) konzentrisch angeordnet ist.

4. Arzneimittelabgabevorrichtung (1) nach einem der Ansprüche 1 bis 3, **dadurch gekennzeichnet, dass** das Zahnrad, das das erste Einheitsrad (14) und das zweite Einheitsrad (15) koppelt, einen ersten Antriebsnocken (19) umfasst, der auf einem Vorsprung (25) angeordnet ist, der sich auf einer zweiten Fläche des ersten Einheitsrads (14) befindet, wobei der erste Antriebsnocken (14) mit einem Sperrradabschnitt (20) des zweiten Einheitsrads (15) zusammenwirkt, wobei der Sperrradabschnitt (20) eine Menge an Zähnen, vorzugsweise zehn Zähne, einschließt.

5. Arzneimittelabgabevorrichtung nach Anspruch 4,
**dadurch gekennzeichnet, dass** das erste Einheitsrad (14) einen ersten Blockierungsnocken (21) umfasst, der derart geformt ist, dass er in eine von mehreren Vertiefungen eines ersten Scheibenbereichs (22) des zweiten Einheitsrads (15) einrastet, wenn der erste Antriebsnocken (19) nicht in einen Zahn des Sperrradabschnitts (20) eingerastet ist und nicht in eine Vertiefung des ersten Scheibenbereichs (22) einrastet, wenn der erste Antriebsnocken (19) in einen Zahn des Sperrradabschnitts (20) einrastet.

6. Arzneimittelabgabevorrichtung (1) nach Anspruch 5,
**dadurch gekennzeichnet, dass** das erste Einheitsrad (14) einen zweiten Blockierungsnocken (23) umfasst, der sich in einem Abstand zu dem ersten Blockierungsnocken (21) auf dem Vorsprung (25) befindet, wobei der zweite Blockierungsnocken (23) derart geformt ist, dass er in eine von einer Menge an Vertiefungen eines zweiten Scheibenbereichs (24) des zweiten Einheitsrads (15) einrastet, wenn der Antriebsnocken (19) nicht in einen Zahn des Sperrradabschnitts (20) einrastet, und in keine Vertiefung des zweiten Scheibenbereichs (24) einrastet, wenn der Antriebsnocken in einen Zahn des Sperrradabschnitts (20) einrastet, wobei der erste Scheibenbereich (22) und der zweite Scheibenbereich (24) derart angeordnet sind, dass der Vorsprung (25) des ersten Einheitsrads (14) sich zwischen dem ersten Scheibenbereich (22) und dem zweiten Scheibenbereich (24) befindet.

7. Arzneimittelabgabevorrichtung (1) nach einem der Ansprüche 1 bis 6, **dadurch gekennzeichnet, dass** der Dosiszähler ein drittes Einheitsrad (16) einschließt, das neben dem zweiten Einheitsrad (15) angeordnet ist und das mit dem zweiten Einheitsrad (15) mittels eines zweiten Zahnrades (36) gekoppelt ist, das für jede komplette Umdrehung des zweiten Einheitsrads (15) eine Teilumdrehung des dritten Einheitsrads (16) ermöglicht, wobei die Teilumdrehung vorzugsweise ein Zehntel einer kompletten Umdrehung beträgt.

8. Arzneimittelabgabevorrichtung (1) nach Anspruch 7,
**dadurch gekennzeichnet, dass** das zweite Zahnrad (36) einen zweiten Antriebsnocken (29), der auf dem zweiten Einheitsrad (15) angeordnet ist, und eine Vielzahl von Kontaktfahnen (41), vorzugsweise zehn Kontaktfahnen (41), umfasst, die auf einer Fläche des dritten Einheitsrads (16) in einer schaufelradartigen Anordnung (28) angeordnet sind, wobei der zweite Antriebsnocken (29) konfiguriert ist, um einmal pro Umdrehung des zweiten Einheitsrads (15) zwischen zwei benachbarte Kontaktfahnen (41) einzurasten.

9. Arzneimittelabgabevorrichtung (1) nach Anspruch 8,
**dadurch gekennzeichnet, dass** das zweite Einheitsrad einen ringförmigen Vorsprung (30) mit einem Spalt (31) einschließt, wobei der Spalt derart bemessen und positioniert ist, dass der ringförmige Vorsprung (30) die schaufelradartige Anordnung (28) von Kontaktfahnen (41) durchquert, wenn der zweite Antriebsnocken (29) in keine der Kontaktfahnen (41) einrastet, und nicht die schaufelradartige Anordnung (28) durchquert, wenn der zweite Antriebsnocken (29) in eine benachbarte Kontaktfahne (41) einrastet.

10. Arzneimittelabgabevorrichtung (1) nach einem der Ansprüche 7 bis 9, **dadurch gekennzeichnet, dass** eine Drehachse des dritten Einheitsrades (16) deckungsgleich mit der Drehachse des ersten Einheitsrades (14) ist und der Durchmesser des dritten Einheitsrades (16) dem Durchmesser des ersten Einheitsrades (14) entspricht.

11. Arzneimittelabgabevorrichtung (1) nach Anspruch 10,
**dadurch gekennzeichnet, dass** das zweite Einheitsrad (15) einen Außenring (32) und einen kleineren konzentrischen Innenring (33) umfasst, wobei der Außenring (32) eine Umfangsoberfläche sowie den ersten Scheibenbereich (22), der auf der Rückseite der Umfangsoberfläche angeordnet ist, einschließt, und wobei der Innenring (33) den Sperrradabschnitt (20), den zweiten Scheibenbereich, den zweiten Antriebsnocken und den ringförmigen Vorsprung (30), die nebeneinander angeordnet sind, umfasst.

12. Arzneimittelabgabevorrichtung (1) nach einem der Ansprüche 1 bis 11, **dadurch gekennzeichnet, dass** die Drehachse des ersten Einheitsrades (14) in radialer Richtung mittig zwischen einer Außenoberfläche des Gewindes (11) der Kolbenstange (10) und einer Innenoberfläche des Gehäuses (2) angeordnet ist.

13. Arzneimittelabgabevorrichtung (1) nach einem der Ansprüche 1 bis 12, **dadurch gekennzeichnet, dass** die Vorrichtung (1) eine stiftförmige Injektionsvorrichtung ist.

## Revendications

1. Dispositif d'administration de médicaments (1) comportant un boîtier (2), une tige de piston (10) avec une surface extérieure filetée, la tige de piston (10) étant destinée à faire avancer un piston (9) à l'intérieur d'un réservoir (8) contenant un médicament afin de distribuer le médicament, la tige de piston (10) étant agencée parallèlement à un axe central du boîtier (2) et de préférence en congruence avec celui-ci, le dispositif d'administration de médicaments (1) comprenant en outre un entraînement (45) pour déplacer la tige de piston (10) soit en rotation, soit de façon linéaire autour de l'axe central,
dans lequel le dispositif d'administration de médicaments (1) comprend en outre un compteur de doses pour indiquer la quantité de médicament distribuée par le réservoir (8) ou restant dans celui-ci, ledit compteur de doses comprenant une première roue unitaire (14) et une deuxième roue unitaire (15) toutes deux agencées l'une à côté de l'autre le long de l'axe central du boîtier (2), la deuxième roue unitaire (15) étant accouplée à la première roue unitaire (14) au moyen d'un engrenage qui traduit un mouvement de rotation continu de la première roue unitaire (14) en une rotation intermittente de la deuxième roue unitaire (15),
dans lequel la première roue unitaire (14) comprend une section de roue droite cylindrique sur une première face,
ladite section de roue droite cylindrique (17) ayant des dents qui s'engrènent avec une roue droite cylindrique (13) qui est agencée de manière concentrique autour de la tige de piston (10),
**caractérisé en ce que** la roue droite cylindrique (13) comprend au moins une saillie (37.1) qui s'engrène avec un filetage (11) de la tige de piston (10) pour transformer une rotation de la tige de piston (10) ou un mouvement linéaire de la tige de piston (10) en un mouvement de rotation de la roue droite cylindrique (13).

2. Dispositif d'administration de médicaments (1) selon la revendication 1, **caractérisé en ce que** la première roue unitaire (14) a un axe de rotation qui est parallèle à l'axe central et un diamètre qui est choisi de telle sorte que la première roue unitaire (14) peut être agencée à côté de la tige de piston (10) à l'intérieur du boîtier (2).

3. Dispositif d'administration de médicaments (1) selon l'une quelconque des revendications 1 ou 2, **caractérisé en ce que** la deuxième roue unitaire (15) est agencée de manière concentrique autour de la tige de piston (10).

4. Dispositif d'administration de médicaments (1) selon l'une quelconque des revendications 1 à 3, **caractérisé en ce que** l'engrenage accouplant la première roue unitaire (14) et la deuxième roue unitaire (15) comprend une première came d'entraînement (19) agencée sur une protubérance (25) située sur une seconde face de la première roue unitaire (14), la première came d'entraînement (14) interagissant avec une section de roue à rochet (20) de la deuxième roue unitaire (15), la section de roue à rochet (20) comportant une multitude de dents, de préférence dix dents.

5. Dispositif d'administration de médicaments selon la revendication 4, **caractérisé en ce que** la première roue unitaire (14) comprend une première came de blocage (21) qui est façonnée de manière à venir en prise avec l'une des multiples indentations d'une première zone de disque (22) de la deuxième roue unitaire (15) lorsque la première came d'entraînement (19) n'est pas en prise avec une dent de la section de roue à rochet (20), et à ne venir en prise avec aucune indentation de la première zone de disque (22) lorsque la première came d'entraînement (19) vient en prise avec une dent de la section de roue à rochet (20).

6. Dispositif d'administration de médicaments (1) selon la revendication 5, **caractérisé en ce que** la première roue unitaire (14) comprend une seconde came de blocage (23) située à une certaine distance de la première came de blocage (21) sur la protubérance (25), la seconde came de blocage (23) étant façonnée de manière à venir en prise avec l'une des multiples indentations d'une seconde zone de disque (24) de la deuxième roue unitaire (15) lorsque la came d'entraînement (19) n'est pas en prise avec une dent de la section de roue à rochet (20), et à ne venir en prise avec aucune indentation de la seconde zone de disque (24) lorsque la came d'entraînement vient en prise avec une dent de la section de roue à rochet (20), dans lequel la première zone de disque (22) et la seconde zone de disque (24) sont agencées de telle sorte que la protubérance (25) de la première roue unitaire (14) est située entre la première zone de disque (22) et la seconde zone de disque (24).

7. Dispositif d'administration de médicaments (1) selon l'une quelconque des revendications 1 à 6, **caractérisé en ce que** le compteur de doses comporte une troisième roue unitaire (16) qui est agencée à côté de la deuxième roue unitaire (15) et qui est accouplée à la deuxième roue unitaire (15) au moyen d'un second engrenage (36) qui permet une rotation partielle de la troisième roue unitaire (16) pour chaque rotation complète de la deuxième roue unitaire (15), ladite rotation partielle étant de préférence un dixième d'une rotation complète.

8. Dispositif d'administration de médicaments (1) selon la revendication 7, **caractérisé en ce que** le second engrenage (36) comprend une seconde came d'entraînement (29) agencée sur la deuxième roue unitaire (15) et une pluralité de pattes de contact (41), de préférence dix pattes de contact (41), agencées sur une face de la troisième roue unitaire (16) dans un agencement en forme de roue à aubes (28), dans lequel la seconde came d'entraînement (29) est conçue pour venir en prise entre deux pattes de contact (41) voisines une fois par révolution de la deuxième roue unitaire (15).

9. Dispositif d'administration de médicaments (1) selon la revendication 8, **caractérisé en ce que** la deuxième roue unitaire comporte une protubérance en forme d'anneau (30) avec un espace (31), l'espace étant dimensionné et positionné de telle sorte que la protubérance en forme d'anneau (30) traverse l'agencement en forme de roue à aubes (28) de pattes de contact (41) lorsque la seconde came d'entraînement (29) n'est en prise avec aucune des pattes de contact (41) et ne traverse pas l'agencement en forme de roue à aubes (28) lorsque la seconde came d'entraînement (29) est en prise avec une patte de contact (41) voisine.

10. Dispositif d'administration de médicaments (1) selon l'une quelconque des revendications 7 à 9, **caractérisé en ce qu'**un axe de rotation de la troisième roue unitaire (16) est congruent avec l'axe de rotation de la première roue unitaire (14) et le diamètre de la troisième roue unitaire (16) correspond au diamètre de la première roue unitaire (14).

11. Dispositif d'administration de médicaments (1) selon la revendication 10, **caractérisé en ce que** la deuxième roue unitaire (15) comprend un anneau extérieur (32) et un anneau intérieur (33) concentrique plus petit, l'anneau extérieur (32) comportant une surface périphérique ainsi que la première zone de disque (22) qui est agencée à l'arrière de la surface périphérique, et dans lequel l'anneau intérieur (33) comprend la section de roue à rochet (20), la seconde zone de disque, la seconde came d'entraînement et la protubérance en forme d'anneau (30) agencées les unes à côté des autres.

12. Dispositif d'administration de médicaments (1) selon l'une quelconque des revendications 1 à 11, **caractérisé en ce que** l'axe de rotation de la première roue unitaire (14) est agencé dans la direction radiale à mi-chemin entre une surface extérieure du filetage (11) de la tige de piston (10) et une surface intérieure du boîtier (2).

13. Dispositif d'administration de médicaments (1) selon l'une quelconque des revendications 1 à 12, **caractérisé en ce que** le dispositif (1) est un dispositif d'injection en forme de stylo.
